# EUROPEAN PATENT APPLICATION

(11) **EP 4 682 247 A1**
(43) Date of publication of application: **21.01.2026**
(21) Application number: 24771229.2
(22) Date of filing: 18.03.2024
(51) Int. Cl.: C12N 5/071, G01N 33/50, A61L 27/36

(54) **METHOD FOR CONSTRUCTING AND CULTURING HAIR FOLLICLE ORGANOIDS, AND HAIR DRUG SCREENING METHOD USING SAME, AND HAIR TRANSPLANT MATERIAL**

(30) Priority: 16.03.2023 KR 20230034499
(71) Applicant: Kangstem Biotech Co., Ltd., Seoul 06179 (KR)
(72) Inventor: KANG, Kyung-Sun, Seoul 06338 (KR); KIM, Minji, Seoul 04026 (KR)
(74) Representative: Mewburn Ellis LLP
(86) International application number: PCT/KR2024/003368
(87) International publication number: WO 2024/191230

(57) **Abstract**

The present invention relates to a technology for constructing and culturing hair follicle organoids for hair drug screening methods and production of hair transplant materials. More specifically, in the present invention, hair follicles were isolated from existing skin organoids derived from human induced pluripotent stem cells and cultured on collagen- and Matrigel-coated culture inserts to observe hair follicle growth and degeneration. The transplantation of the cultured hair follicles into the skin resulted in the growth of new hair, confirming a potential use of the hair follicle organoids as hair transplant materials. A hair loss-mimic model was generated on the basis of observing reductions in hair follicle length, damage to hair papilla cell aggregates, decreased expression of WNT3A or β-catenin, and increased expression of DKK-1 or TGF-β2 according to treatment of the hair follicle organoids with alopecia-inducing factors. Through the observation, the organoid is expected to find wide applications such as screening methods for hair growth promoters, alopecia preventatives, alopecia mitigators, or alopecia treatments, and screening methods for cosmetic compositions for promoting hair growth, preventing alopecia, or mitigating alopecia.

## Description

### Technical Field

The present disclosure provides a method of producing and culturing a hair follicle organoid, a hair-related drug screening method using the same, and a hair transplant material.

### Background Art

Human hair is considered one of the most important aspects of human appearance. Human hair is very important not only because it primarily serves to protect the skin and scalp, but also it plays a unique role in social and sexual communication. Alopecia refers to a hairless state in an area where hair should normally exist, or a natural loss of hair that has stopped growing, and it generally means the loss of terminal hair (thick, black hair) on the scalp. About 50 to 100 hairs usually fall out a day, and the greater the number of hairs lost, the more likely to have alopecia.

Many studies on alopecia are being conducted in Korea, and recently, research on many regulatory factors involved in mechanisms of hair growth and hair loss is being actively on the way. Despite the diverse range of hair growth products currently on the market, most of them offer minimal or temporary effects on hair loss prevention and hair growth, leaving consumer expectations unmet. In addition, there is insufficient evidence regarding the efficacy or safety of the products, with serious side effects reported such as recurrence of alopecia upon termination of using the product as well as or sexual dysfunction, causing difficulty in application.

In addition to development of treatments to overcome alopecia, hair transplantation may be considered, where hair transplantation is applied to people with little hair (fur) to artificially implant hair to prevent stress and reduced social adaptability caused by hair, and most importantly, it aims to help the mental health of hair transplant recipients.

These hair transplantations include a method of transplanting natural hair and a method of transplanting artificial hair, and in the case of natural hair transplant, some of the one's own hair is collected to transplant hair roots (hair follicles) to an area without hair so as to induce hair growth in the future.

Although this type of hair transplant has the advantage of less bleeding and scarring as the hair roots are separated one by one to be transplanted to the desired spot in addition to a high degree of determining a direction and location for the hair to grow, it takes a considerable amount of time since the procedure involves separation of hair roots one by one and may cause side effects such as pain and inflammation due to separation, thereby bearing disadvantages in that it requires considerable experience.

In particular, in the case of hair transplantation, since one's own hair is used for transplantation, considering characteristics of hair transplant recipients who have insufficient hair (fur), a difficulty in collection of a large number of hair roots makes it hard to cover a large area by transplantation, and there are also cases where there is a shortage of hair to be transplanted, which has limitations in transplantation with one's own hair. In addition, there has been a time limit in that plucked hair had to be transplanted in real time due to difficulty in storage.

Therefore, there is a need to develop a screening method for discovering a candidate substance for a hair growth agent, anti-alopecia agent, alopecia relief agent, or alopecia treatment agent with a structure similar to an actual hair follicle while mimicking functions to be used as a hair follicle transplant material, or a method of screening a cosmetic composition for promoting hair growth, preventing alopecia, or alleviating alopecia.

### Disclosure of the Invention

### Technical Goals

An object of the present disclosure is to provide a method of preparing a hair follicle organoid, including the following steps of:
(1) separating hair follicles from a skin organoid; and (2) culturing the separated hair follicles at an air-liquid interface.

Another object of the present disclosure is to provide a hair follicle organoid prepared by the preparation method.

Another object of the present disclosure is to provide a hair transplant material including the hair follicle organoid.

Another object of the present disclosure is to provide a method of preparing a strip-like hair follicle organoid, including the following steps of:
(1) planar sectioning a skin organoid and spreading it, followed by vertical cutting; and (2) culturing the cut skin organoid at an air-liquid interface.

Another object of the present disclosure is to provide a strip-like hair follicle organoid prepared by the preparation method.

Another object of the present disclosure is to provide a hair transplant material including the strip-like hair follicle organoid.

Another object of the present disclosure is to provide a method of preparing a hair follicle organoid for an alopecia-mimicking model, including the following steps of:
(1) separating hair follicles from a skin organoid; (2) culturing the separated hair follicles at an air-liquid interface to produce a hair follicle organoid; and (3) treating the hair follicle organoid with an alopecia-inducing factor.

Another object of the present disclosure is to provide a hair follicle organoid for an alopecia-mimicking model produced by the preparation method.

Another object of the present disclosure is to provide a method of preparing a strip-like hair follicle organoid for an alopecia-mimicking model, including the following steps of:
(1) planar sectioning a skin organoid and spreading it, followed by vertical cutting; (2) culturing the cut skin organoid at an air-liquid interface to produce a strip-like hair follicle organoid; and (3) treating the strip-like hair follicle organoid with an alopecia-inducing factor.

Another object of the present disclosure is to provide a strip-like hair follicle organoid for an alopecia-mimicking model produced by the preparation method.

Another object of the present disclosure is to provide a method of screening a hair growth promoter, anti-alopecia agent, alopecia relief agent, or alopecia treatment agent, including the following steps of:
(1) separating hair follicles from a skin organoid; (2) culturing the separated hair follicles at an air-liquid interface to produce a hair follicle organoid; (3) treating the hair follicle organoid with an alopecia-inducing factor to produce a hair follicle organoid for an alopecia-mimicking model; and (4) treating the hair follicle organoid for the alopecia-mimicking model with a candidate substance for a hair growth promoter, anti-alopecia agent, alopecia relief agent, or alopecia treatment agent.

Another object of the present disclosure is to provide a method of screening a hair growth promoter, anti-alopecia agent, alopecia relief agent, or alopecia treatment agent, including the following steps of:
(1) planar sectioning a skin organoid and spreading it, followed by vertical cutting; (2) culturing the cut skin organoid at an air-liquid interface to produce a strip-like hair follicle organoid; (3) treating the strip-like hair follicle organoid with an alopecia-inducing factor to produce a strip-like hair follicle organoid for an alopecia-mimicking model; and (4) treating the strip-like hair follicle organoid for the alopecia-mimicking model with a candidate substance for a hair growth promoter, anti-alopecia agent, alopecia relief agent, or alopecia treatment agent.

Another object of the present disclosure is to provide a method of screening a hair growth promoter, anti-alopecia agent, alopecia relief agent, or alopecia treatment agent, including the following steps of:
(1) separating hair follicles from a skin organoid; (2) culturing the separated hair follicles at an air-liquid interface to produce a hair follicle organoid; and (3) treating the hair follicle organoid with a candidate substance for a hair growth promoter, anti-alopecia agent, alopecia relief agent, or alopecia treatment agent.

Another object of the present disclosure is to provide a method of screening a hair growth promoter, anti-alopecia agent, alopecia relief agent, or alopecia treatment agent, including the following steps of:
(1) planar sectioning a skin organoid and spreading it, followed by vertical cutting; (2) culturing the cut skin organoid at an air-liquid interface to produce a strip-like hair follicle organoid; and (3) treating the strip-like hair follicle organoid with a candidate substance for a hair growth promoter, anti-alopecia agent, alopecia relief agent, or alopecia treatment agent.

Another object of the present disclosure is to provide a method of screening a cosmetic composition for promoting hair growth, preventing alopecia, or alleviating alopecia, including the following steps of:
(1) separating hair follicles from a skin organoid; (2) culturing the separated hair follicles at an air-liquid interface to produce a hair follicle organoid; (3) treating the hair follicle organoid with an alopecia-inducing factor to produce a hair follicle organoid for an alopecia-mimicking model; and (4) treating the hair follicle organoid for the alopecia-mimicking model with a candidate substance for a cosmetic composition for promoting hair growth, preventing alopecia, or alleviating alopecia.

Another object of the present disclosure is to provide a method of screening a cosmetic composition for promoting hair growth, preventing alopecia, or alleviating alopecia, including the following steps of:
(1) planar sectioning a skin organoid and spreading it, followed by vertical cutting; (2) culturing the cut skin organoid at an air-liquid interface to produce a strip-like hair follicle organoid; (3) treating the strip-like hair follicle organoid with an alopecia-inducing factor to produce a stirp-like hair follicle organoid for an alopecia-mimicking model; and (4) treating the stirp-like hair follicle organoid for an alopecia-mimicking model with a candidate substance for a cosmetic composition for promoting hair growth, preventing alopecia, or alleviating alopecia.

Another object of the present disclosure is to provide a method of screening a cosmetic composition for promoting hair growth, preventing alopecia, or alleviating alopecia, including the following steps of:
(1) separating hair follicles from a skin organoid; (2) culturing the separated hair follicles at an air-liquid interface to produce a hair follicle organoid; and (3) treating the hair follicle organoid with a candidate substance for a cosmetic composition for promoting hair growth, preventing alopecia, or alleviating alopecia.

Another object of the present disclosure is to provide a method of screening a cosmetic composition for promoting hair growth, preventing alopecia, or alleviating alopecia, including the following steps of:
(1) planar sectioning a skin organoid and spreading it, followed by vertical cutting; (2) culturing the cut skin organoid at an air-liquid interface to produce a strip-like hair follicle organoid; and (3) treating the strip-like hair follicle organoid with a candidate substance for a cosmetic composition for promoting hair growth, preventing alopecia, or alleviating alopecia.

### Technical Solutions

To achieve the above objects, the present disclosure provides a method of preparing a hair follicle organoid, including the following steps of:
(1) separating hair follicles from a skin organoid; and (2) culturing the separated hair follicles at an air-liquid interface.

In addition, the present disclosure provides a hair follicle organoid prepared by the preparation method.

In addition, the present disclosure provides a hair transplant material including the hair follicle organoid.

In addition, the present disclosure provides a method of preparing a strip-like hair follicle organoid, including the following steps of:
(1) planar sectioning a skin organoid and spreading it, followed by vertical cutting; and (2) culturing the cut skin organoid at an air-liquid interface.

In addition, the present disclosure provides a strip-like hair follicle organoid prepared by the preparation method.

In addition, the present disclosure provides a hair transplant material including the strip-like hair follicle organoid.

In addition, the present disclosure provides a method of preparing a hair follicle organoid for an alopecia-mimicking model, including the following steps of:
(1) separating hair follicles from a skin organoid; (2) culturing the separated hair follicles at an air-liquid interface to produce a hair follicle organoid; and (3) treating the hair follicle organoid with an alopecia-inducing factor.

In addition, the present disclosure provides a hair follicle organoid for an alopecia-mimicking model produced by the preparation method.

In addition, the present disclosure provides a method of preparing a strip-like hair follicle organoid for an alopecia-mimicking model, including the following steps of:
(1) planar sectioning a skin organoid and spreading it, followed by vertical cutting; (2) culturing the cut skin organoid at an air-liquid interface to produce a strip-like hair follicle organoid; and (3) treating the strip-like hair follicle organoid with an alopecia-inducing factor.

In addition, the present disclosure provides a strip-like hair follicle organoid for an alopecia-mimicking model produced by the preparation method.

In addition, the present disclosure provides a method of screening a hair growth promoter, anti-alopecia agent, alopecia relief agent, or alopecia treatment agent, including the following steps of:
(1) separating hair follicles from a skin organoid; (2) culturing the separated hair follicles at an air-liquid interface to produce a hair follicle organoid; (3) treating the hair follicle organoid with an alopecia-inducing factor to produce a hair follicle organoid for an alopecia-mimicking model; and (4) treating the hair follicle organoid for the alopecia-mimicking model with a candidate substance for a hair growth promoter, anti-alopecia agent, alopecia relief agent, or alopecia treatment agent.

In addition, the present disclosure provides a method of screening a hair growth promoter, anti-alopecia agent, alopecia relief agent, or alopecia treatment agent, including the following steps of:
(1) planar sectioning a skin organoid and spreading it, followed by vertical cutting; (2) culturing the cut skin organoid at an air-liquid interface to produce a strip-like hair follicle organoid; (3) treating the strip-like hair follicle organoid with an alopecia-inducing factor to produce a strip-like hair follicle organoid for an alopecia-mimicking model; and (4) treating the strip-like hair follicle organoid for the alopecia-mimicking model with a candidate substance for a hair growth promoter, anti-alopecia agent, alopecia relief agent, or alopecia treatment agent.

In addition, the present disclosure provides a method of screening a hair growth promoter, anti-alopecia agent, alopecia relief agent, or alopecia treatment agent, including the following steps of:
(1) separating hair follicles from a skin organoid; (2) culturing the separated hair follicles at an air-liquid interface to produce a hair follicle organoid; and (3) treating the hair follicle organoid with a candidate substance for a hair growth promoter, anti-alopecia agent, alopecia relief agent, or alopecia treatment agent.

In addition, the present disclosure provides a method of screening a hair growth promoter, anti-alopecia agent, alopecia relief agent, or alopecia treatment agent, including the following steps of:
(1) planar sectioning a skin organoid and spreading it, followed by vertical cutting; (2) culturing the cut skin organoid at an air-liquid interface to produce a strip-like hair follicle organoid; and (3) treating the strip-like hair follicle organoid with a candidate substance for a hair growth promoter, anti-alopecia agent, alopecia relief agent, or alopecia treatment agent.

In addition, the present disclosure provides a method of screening a cosmetic composition for promoting hair growth, preventing alopecia, or alleviating alopecia, including the following steps of:
(1) separating hair follicles from a skin organoid; (2) culturing the separated hair follicles at an air-liquid interface to produce a hair follicle organoid; (3) treating the hair follicle organoid with an alopecia-inducing factor to produce a hair follicle organoid for an alopecia-mimicking model; and (4) treating the hair follicle organoid for the alopecia-mimicking model with a candidate substance for a cosmetic composition for promoting hair growth, preventing alopecia, or alleviating alopecia.

In addition, the present disclosure provides a method of screening a cosmetic composition for promoting hair growth, preventing alopecia, or alleviating alopecia, including the following steps of:
(1) planar sectioning a skin organoid and spreading it, followed by vertical cutting; (2) culturing the cut skin organoid at an air-liquid interface to produce a strip-like hair follicle organoid; (3) treating the strip-like hair follicle organoid with an alopecia-inducing factor to produce a stirp-like hair follicle organoid for an alopecia-mimicking model; and (4) treating the stirp-like hair follicle organoid for an alopecia-mimicking model with a candidate substance for a cosmetic composition for promoting hair growth, preventing alopecia, or alleviating alopecia.

In addition, the present disclosure provides a method of screening a cosmetic composition for promoting hair growth, preventing alopecia, or alleviating alopecia, including the following steps of:
(1) separating hair follicles from a skin organoid; (2) culturing the separated hair follicles at an air-liquid interface to produce a hair follicle organoid; and (3) treating the hair follicle organoid with a candidate substance for a cosmetic composition for promoting hair growth, preventing alopecia, or alleviating alopecia.

In addition, the present disclosure provides a method of screening a cosmetic composition for promoting hair growth, preventing alopecia, or alleviating alopecia, including the following steps of:
(1) planar sectioning a skin organoid and spreading it, followed by vertical cutting; (2) culturing the cut skin organoid at an air-liquid interface to produce a strip-like hair follicle organoid; and (3) treating the strip-like hair follicle organoid with a candidate substance for a cosmetic composition for promoting hair growth, preventing alopecia, or alleviating alopecia.

### Advantageous Effects

The present disclosure relates to a hair-related drug screening method and a technique for producing and culturing a hair follicle organoid for production of a hair transplant material and, more specifically, the growth and degeneration of hair follicles were identified by separating hair follicles at a hair peg stage from an existing skin organoid using a human induced pluripotent stem cell line, followed by culture on a culture insert coated with collagen and Matrigel, and the growth of new hair was observed as a result of transplanting the cultured hair follicles to the skin, such that it is expected to be utilized as a hair transplant material.

In addition, upon treatment of the hair follicle organoid of the present disclosure with an alopecia-inducing factor, a decrease in a length of hair follicles, damage to hair papilla cell aggregates, decreased expression of WNT3A or β-catenin, and increased expression of DKK-1 or TGF-β2 were observed to lead to creation of an alopecia-mimicking model, such that it is expected that the present disclosure may be utilized as a method of screening a hair growth promoter, anti-alopecia agent, alopecia relief agent, or alopecia treatment agent, or a method of screening a cosmetic composition for promoting hair growth, preventing alopecia, or alleviating alopecia.

### Brief Description of Drawings

FIG. 1 shows identification of development of a skin organoid and generation of hair follicles using a human induced pluripotent stem cell line.
FIG. 2 shows identification of structural characteristics of hair follicles observed in a produced skin organoid.
FIG. 3 shows identification of a hair follicle at a hair germ stage or a hair peg stage.
FIG. 4 shows identification of culture results for hair follicles separated from a produced skin organoid at the hair germ stage or the hair peg stage.
FIG. 5 shows results of culturing hair follicles at an air-liquid interface after producing an artificial extracellular matrix, which is a coating support on a culture insert, under various conditions (collagen only, Matrigel only, collagen and Matrigel in combination).
FIG. 6 shows identification of growth and degeneration of hair follicles by observing changes in expression of DKK-1, β-catenin, and CD34 genes.
FIG. 7 shows identification of in-vivo transplantation results for a hair follicle organoid.
FIG. 8 shows identification of changes in a boundary of hair papilla cell aggregates, a length of hair follicles, and expression of WNT3A and β-catenin genes following treatment of a hair follicle organoid with dihydrotestosterone (DHT) and minoxidil (MXD).
FIG. 9 shows identification of changes in a length of hair follicles and hair depending on cell death of a strip-like hair follicle organoid, presence of cell proliferation, and treatment with alopecia-inducing hormones.

### Best Mode for Carrying Out the Invention

Hereinafter, the present disclosure will be described in more detail.

The present disclosure provides a method of preparing a hair follicle organoid, including the following steps of: (1) separating hair follicles from a skin organoid; and (2) culturing the separated hair follicles at an air-liquid interface.

The skin organoid in the step (1) may be prepared by including the following steps of:

(a) culturing a pluripotent stem cell-derived organoid in the presence of a Wnt agonist; (b) culturing the culture of the step (a) in a medium for skin organoid maturation for at least 40 days; and (c) cutting the culture of the step (b) followed by culture at an air-liquid interface.

The Wnt agonist may be added on day 5 to day 7 of culturing the pluripotent stem cells.

The Wnt agonist may be CHIR-99021.

The skin organoid may express one or more selected from the group consisting of keratin 5 (KRT5), KRT10, KRT15, KRT17, loricrin, filaggrin, SRY-box transcription factor 2 (SOX2), and Melan-A, but is not limited thereto.

The hair follicle of the step (1) may include any one or more selected from the group consisting of a dermal sheath, an outer sheath, an inner sheath, a hair papilla cell, a hair follicle cell, a bulge area, and a sebaceous gland, but is not limited thereto.

The hair follicle in the step (1) may be separated at a hair germ stage or a hair peg stage.

The hair germ stage may be formed within 60 to 80 days after formation of the skin organoid, and preferably within 70 to 80 days.

The hair peg stage may be formed within 80 to 100 days after formation of the skin organoid, and preferably within 90 to 100 days.

The separation in the step (1) may be performed using microdissection.

The culturing in the step (2) may be performed on a culture insert.

The culture insert may be coated with collagen and Matrigel; or collagen.

The collagen and Matrigel may be in a ratio of 1:10 to 10:1, preferably 1:1 to 3:1.

In addition, the present disclosure provides a hair follicle organoid prepared by the preparation method.

The hair follicle organoid may express one or more selected from the group consisting of keratin 5 (KRT5), KRT10, KRT15, KRT17, loricrin, filaggrin, SRY-box transcription factor 2 (SOX2), and Melan-A, but is not limited thereto.

In addition, the present disclosure provides a hair transplant material including the hair follicle organoid.

In addition, the present disclosure provides a method of preparing a strip-like hair follicle organoid, including the following steps of:
(1) planar sectioning a skin organoid and spreading it, followed by vertical cutting; and (2) culturing the cut skin organoid at an air-liquid interface.

The skin organoid of the step (1) may be prepared by including the following steps of:
(a) culturing a pluripotent stem cell-derived organoid in the presence of a Wnt agonist; (b) culturing the culture of the step (a) in a medium for skin organoid maturation for at least 40 days; and (c) cutting the culture of the step (b) followed by culture at air-liquid interface.

The Wnt agonist may be added on day 5 to day 7 of culturing the pluripotent stem cells.

The Wnt agonist may be CHIR-99021.

The skin organoid may express one or more selected from the group consisting of keratin 5 (KRT5), KRT10, KRT15, KRT17, loricrin, filaggrin, SRY-box transcription factor 2 (SOX2), and Melan-A, but is not limited thereto.

The cutting in the step (1) may be performed when the hair follicle in the skin organoid is in the hair germ stage or the hair peg stage.

The hair germ stage may be formed within 60 to 80 days after formation of the skin organoid, and preferably within 70 to 80 days.

The hair peg stage may be formed within 80 to 100 days after formation of the skin organoid, and preferably within 90 to 100 days.

The cutting in the step (1) may be carried out by cutting the spread skin organoid at intervals of 1 mm to 3 mm.

In addition, the present disclosure provides a strip-like hair follicle organoid prepared by the preparation method.

The strip-like hair follicle organoid may express one or more selected from the group consisting of keratin 5 (KRT5), KRT10, KRT15, KRT17, loricrin, filaggrin, SRY-box transcription factor 2 (SOX2), and Melan-A, but is not limited thereto.

In addition, the present disclosure provides a hair transplant material including the strip-like hair follicle organoid.

In addition, the present disclosure provides a method of preparing a hair follicle organoid for an alopecia-mimicking model, including the following steps of:
(1) separating hair follicles from a skin organoid; (2) culturing the separated hair follicles at an air-liquid interface to produce a hair follicle organoid; and (3) treating the hair follicle organoid with an alopecia-inducing factor.

The skin organoid of the step (1) may be prepared by including the following steps of:
(a) culturing a pluripotent stem cell-derived organoid in the presence of a Wnt agonist; (b) culturing the culture of the step (a) in a medium for skin organoid maturation for at least 40 days; and (c) cutting the culture of the step (b) followed by culture at an air-liquid interface.

The Wnt agonist may be added on day 5 to day 7 of culturing the pluripotent stem cells.

The Wnt agonist may be CHIR-99021.

The skin organoid may express one or more selected from the group consisting of keratin 5 (KRT5), KRT10, KRT15, KRT17, loricrin, filaggrin, SRY-box transcription factor 2 (SOX2), and Melan-A, but is not limited thereto.

The alopecia may be caused by hormonal imbalance or ultraviolet rays.

The hormones may be dihydrotestosterone (DHT), testosterone, thyroid hormones, or cortisol.

The thyroid hormone may be tyrosine.

The alopecia-inducing factor in the step (3) may be male hormones, stress hormones, or ultraviolet rays.

The hormone may be, but is not limited to, dihydrotestosterone, testosterone, or thyroid hormone.

The stress hormone may be cortisol.

The thyroid hormone may be tyrosine.

In addition, the present disclosure provides a hair follicle organoid for an alopecia-mimicking model produced by the preparation method.

The hair follicle organoid for the alopecia-mimicking model may have any one or more characteristics selected from, but not limited to, the group consisting of:
1) reduction in a length of hair follicles; 2) damage to hair papillary cell aggregates; 3) decreased expression of WNT3A or β-catenin; or 4) increased expression of DKK-1 or TGF-β2.

In addition, the present disclosure provides a method of preparing a strip-like hair follicle organoid for an alopecia-mimicking model, including the following steps of:
(1) planar sectioning a skin organoid and spreading it, followed by vertical cutting; (2) culturing the cut skin organoid at an air-liquid interface to produce a strip-like hair follicle organoid; and (3) treating the strip-like hair follicle organoid with an alopecia-inducing factor.

The skin organoid of the step (1) may be prepared by including the following steps of:
(a) culturing a pluripotent stem cell-derived organoid in the presence of a Wnt agonist; (b) culturing the culture of the step (a) in a medium for skin organoid maturation for at least 40 days; and (c) cutting the culture of the step (b) followed by culture at an air-liquid interface.

The skin organoid may express one or more selected from the group consisting of keratin 5 (KRT5), KRT10, KRT15, KRT17, loricrin, filaggrin, SRY-box transcription factor 2 (SOX2), and Melan-A, but is not limited thereto.

The cutting in the step (1) may be performed when the hair follicle in the skin organoid is in the hair germ stage or the hair peg stage.

The alopecia-inducing factor in the step (3) may be male hormones, stress hormones, or ultraviolet rays.

In addition, the present disclosure provides a strip-like hair follicle organoid for an alopecia-mimicking model produced by the preparation method.

The strip-like hair follicle organoid for the alopecia-mimicking model may have any one or more characteristics selected from, but not limited to, the group consisting of:
1) reduction in a length of hair follicles; 2) damage to hair papillary cell aggregates; 3) decreased expression of WNT3A or β-catenin; or 4) increased expression of DKK-1 or TGF-β2.

In addition, the present disclosure provides a method of screening a hair growth promoter, anti-alopecia agent, alopecia relief agent, or alopecia treatment agent, including the following steps of:
(1) separating hair follicles from a skin organoid; (2) culturing the separated hair follicles at an air-liquid interface to produce a hair follicle organoid; (3) treating the hair follicle organoid with an alopecia-inducing factor to produce a hair follicle organoid for an alopecia-mimicking model; and (4) treating the hair follicle organoid for the alopecia-mimicking model with a candidate substance for a hair growth promoter, anti-alopecia agent, alopecia relief agent, or alopecia treatment agent.

The method may include, if expression of WNT3A or β-catenin increases or expression of DKK-1 or TGF-β2 decreases after treatment of the candidate substance in the step (4), determining the candidate substance as a hair growth promoter, anti-alopecia agent, alopecia relief agent, or alopecia treatment agent.

In addition, the present disclosure provides a method of screening a hair growth promoter, anti-alopecia agent, alopecia relief agent, or alopecia treatment agent, including the following steps of:
(1) planar sectioning a skin organoid and spreading it, followed by vertical cutting; (2) culturing the cut skin organoid at an air-liquid interface to produce a strip-like hair follicle organoid; (3) treating the strip-like hair follicle organoid with an alopecia-inducing factor to produce a strip-like hair follicle organoid for an alopecia-mimicking model; and (4) treating the strip-like hair follicle organoid for the alopecia-mimicking model with a candidate substance for a hair growth promoter, anti-alopecia agent, alopecia relief agent, or alopecia treatment agent.

The method may include, if expression of WNT3A or β-catenin increases or expression of DKK-1 or TGF-β2 decreases after treatment of the candidate substance in the step (4), determining the candidate substance as a hair growth promoter, anti-alopecia agent, alopecia relief agent, or alopecia treatment agent.

In addition, the present disclosure provides a method of screening a hair growth promoter, anti-alopecia agent, alopecia relief agent, or alopecia treatment agent, including the following steps of:
(1) separating hair follicles from a skin organoid; (2) culturing the separated hair follicles at an air-liquid interface to produce a hair follicle organoid; and (3) treating the hair follicle organoid with a candidate substance for a hair growth promoter, anti-alopecia agent, alopecia relief agent, or alopecia treatment agent.

The method may include, if expression of WNT3A or β-catenin increases or expression of DKK-1 or TGF-β2 decreases after treatment of the candidate substance in the step (3), determining the candidate substance as a hair growth promoter, anti-alopecia agent, alopecia relief agent, or alopecia treatment agent.

In addition, the present disclosure provides a method of screening a hair growth promoter, anti-alopecia agent, alopecia relief agent, or alopecia treatment agent, including the following steps of:
(1) planar sectioning a skin organoid and spreading it, followed by vertical cutting; (2) culturing the cut skin organoid at an air-liquid interface to produce a strip-like hair follicle organoid; and (3) treating the strip-like hair follicle organoid with a candidate substance for a hair growth promoter, anti-alopecia agent, alopecia relief agent, or alopecia treatment agent.

The method may include, if expression of WNT3A or β-catenin increases or expression of DKK-1 or TGF-β2 decreases after treatment of the candidate substance in the step (3), determining the candidate substance as a hair growth promoter, anti-alopecia agent, alopecia relief agent, or alopecia treatment agent.

In addition, the present disclosure provides a method of screening a cosmetic composition for promoting hair growth, preventing alopecia, or alleviating alopecia, including the following steps of:
(1) separating hair follicles from a skin organoid; (2) culturing the separated hair follicles at an air-liquid interface to produce a hair follicle organoid; (3) treating the hair follicle organoid with an alopecia-inducing factor to produce a hair follicle organoid for an alopecia-mimicking model; and (4) treating the hair follicle organoid for the alopecia-mimicking model with a candidate substance for a cosmetic composition for promoting hair growth, preventing alopecia, or alleviating alopecia.

The method may include, if expression of WNT3A or β-catenin increases or expression of DKK-1 or TGF-β2 decreases after treatment of the candidate substance in the step (4), determining the candidate substance as a cosmetic composition for promoting hair growth, preventing alopecia, or alleviating alopecia.

In addition, the present disclosure provides a method of screening a cosmetic composition for promoting hair growth, preventing alopecia, or alleviating alopecia, including the following steps of:
(1) planar sectioning a skin organoid and spreading it, followed by vertical cutting; (2) culturing the cut skin organoid at an air-liquid interface to produce a strip-like hair follicle organoid; (3) treating the strip-like hair follicle organoid with an alopecia-inducing factor to produce a stirp-like hair follicle organoid for an alopecia-mimicking model; and (4) treating the stirp-like hair follicle organoid for an alopecia-mimicking model with a candidate substance for a cosmetic composition for promoting hair growth, preventing alopecia, or alleviating alopecia.

The method may include, if expression of WNT3A or β-catenin increases or expression of DKK-1 or TGF-β2 decreases after treatment of the candidate substance in the step (4), determining the candidate substance as a cosmetic composition for promoting hair growth, preventing alopecia, or alleviating alopecia.

In addition, the present disclosure provides a method of screening a cosmetic composition for promoting hair growth, preventing alopecia, or alleviating alopecia, including the following steps of:
(1) separating hair follicles from a skin organoid; (2) culturing the separated hair follicles at an air-liquid interface to produce a hair follicle organoid; and (3) treating the hair follicle organoid with a candidate substance for a cosmetic composition for promoting hair growth, preventing alopecia, or alleviating alopecia.

The method may include, if expression of WNT3A or β-catenin increases or expression of DKK-1 or TGF-β2 decreases after treatment of the candidate substance in the step (3), determining the candidate substance as a cosmetic composition for promoting hair growth, preventing alopecia, or alleviating alopecia.

In addition, the present disclosure provides a method of screening a cosmetic composition for promoting hair growth, preventing alopecia, or alleviating alopecia, including the following steps of:
(1) planar sectioning a skin organoid and spreading it, followed by vertical cutting; (2) culturing the cut skin organoid at an air-liquid interface to produce a strip-like hair follicle organoid; and (3) treating the strip-like hair follicle organoid with a candidate substance for a cosmetic composition for promoting hair growth, preventing alopecia, or alleviating alopecia.

The method may include, if expression of WNT3A or β-catenin increases or expression of DKK-1 or TGF-β2 decreases after treatment of the candidate substance in the step (3), determining the candidate substance as a cosmetic composition for promoting hair growth, preventing alopecia, or alleviating alopecia.

Hereinafter, the present disclosure will be described in more detail through examples to help understanding of the present disclosure. However, examples below are merely intended to illustrate the present disclosure, and the scope of the present disclosure is not limited to the following examples. Examples of the present disclosure are provided to more completely explain the present disclosure to those skilled in the art.

### Modes for Carrying Out the Invention

### <Example 1> Production of skin organoid derived from human induced pluripotent stem cells

### 1. Culture of human induced pluripotent stem cells

Human induced pluripotent stem cell lines (iPSC; CMC3, CMC11) were cultured on Vitronectin (ThermoFisher)-coated culture dishes in Essential 8 (Gibo) medium supplemented with Y-27632 (10 µM). The culture medium was replaced daily, and subculture was performed using ReLeSR (Stem Cell Technology) at cycles of approximately 4 days.

### 2. Method of differentiating skin organoids using a human induced pluripotent stem cell line

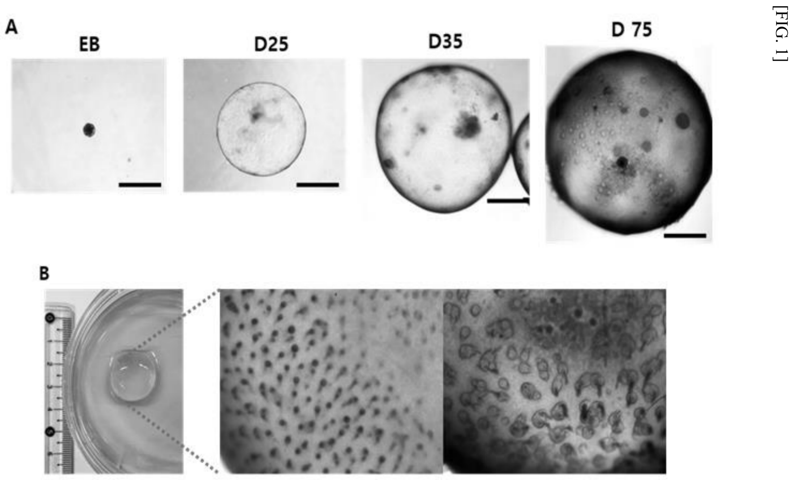

The skin organoid was cultured with reference to existing skin organoid differentiation protocols using human induced pluripotent stem cell lines, disclosed in documents and patents (Jung, S. et al. Wnt-activating human skin organoid model of atopic dermatitis induced by Staphylococcus aureus and its protective effects by Cutibacterium acnes. iScience, 25(10), 105150(2022), METHOD FOR CONSTRUCTION OF ATOPIC DERMATITS MODEL BY USING PLURIPOTENT STEM CELL-DERIVED SKIN ORGANOID, PCT/KR2021/014810 (2021.10.21.)). Skin organoids were cultured from human induced pluripotent stem cells by undergoing steps of 1) culturing embryoid bodies, 2) inducing differentiation into non-neural ectoderm, and 3) inducing differentiation into cranial neural crest-like cells and activating Wnt signals (FIG. 1A).

In skin organoids, it was found that approximately 10 hair follicles were developed per 1 mm² area. (Typically, approximately 45,000 identical hair follicles develop from a single spherical skin organoid) (FIG. 1B).

### 3. Characterization of hair follicles from the skin organoid

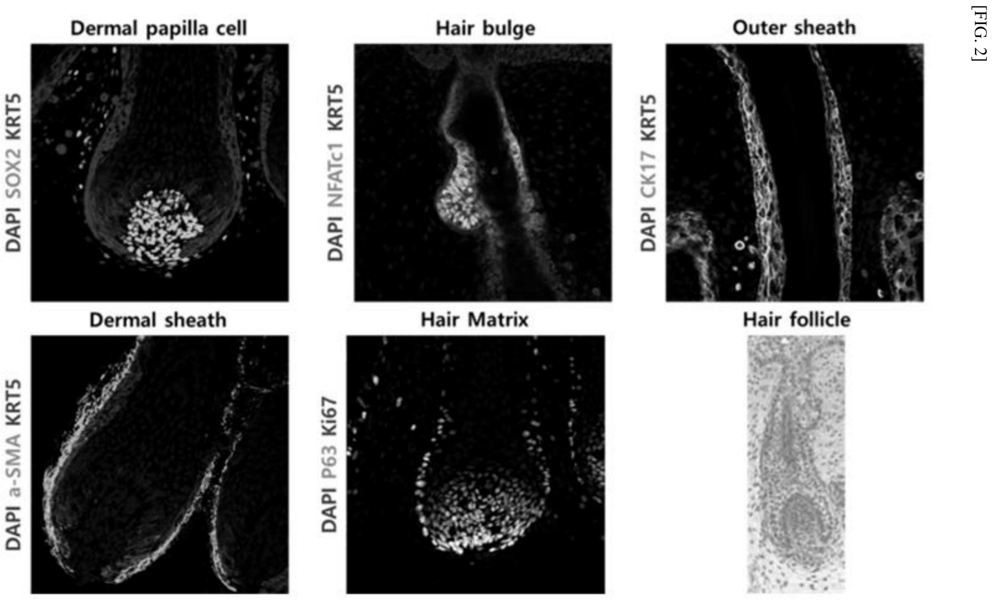

To verify the structural characteristics of the hair follicles shown in the produced skin organoids, H&E and immunofluorescence staining were performed, and the microstructures of the hair follicles, including the dermal sheath, outer sheath, inner sheath, hair papilla cells, hair follicle cells, bulge area, and sebaceous glands, were all identified (FIG. 2).

### <Example 2> Separation and culture of hair follicles from human induced pluripotent stem cell-derived skin organoids

### 1. Separation and culture via microdissection

In human induced pluripotent stem cell-derived skin organoids, hair follicles were separated from the epidermal and dermal layers. First, because hair follicles exist in very small sizes in skin organoids, a microdissection technique was developed to obtain hair follicles without damage.

The skin organoid was transferred to a tissue culture plate and then cut out using Spring Micro-Scissors, and the target tissue was separated from the epidermal and dermal layers using forceps. Due to a tendency of being highly embedded and tightly adhering to the collagen fibers of the hair follicles and skin layers (epidermis and dermis), the target tissues were carefully removed with microneedles. The separated hair follicles were washed with culture medium to avoid contamination from other cells and then cultured on transwell culture inserts coated in 3D with collagen with an exposure to air.

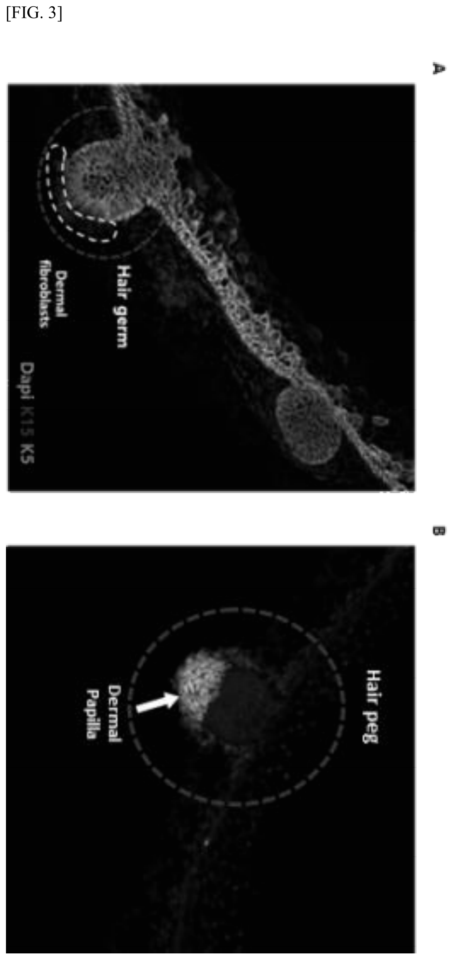

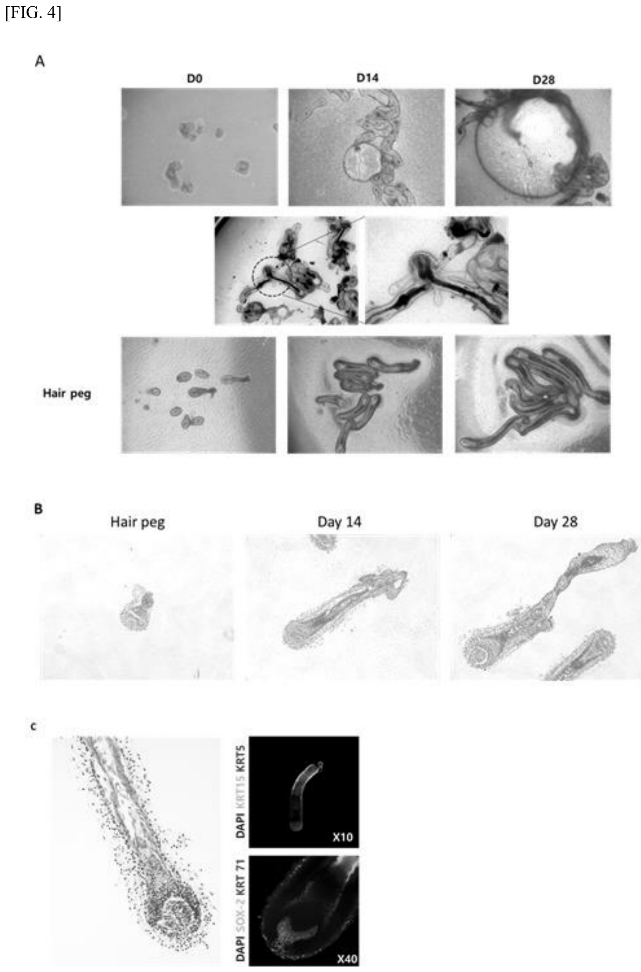

To optimize an appropriate separation period, hair follicles were separated at different stages: a hair germ stage (a stage where the epidermis thickens and protrudes downward, and dermal fibroblasts condense beneath the hair placode (FIG. 3A)); and a hair peg stage (a stage where the end elongates into a rounded columnar shape, dermal fibroblasts form hair papilla cells that resemble bulbs, and the concave proximal end begins to surround the condensed cells (FIG. 3B)). When separated during the hair germ stage, there was a difficulty in completely separating the epidermal layer and the dermal layer, resulting in the regeneration of the epidermal layer and the dermal layer during culture. In addition, it was found that when hair follicles before the hair peg stage were separated and cultured, new hair follicles were formed at the upper part of the hair follicles. On the other hand, when hair follicles after the hair peg stage were separated, it was found that hair grew from perfectly structured hair follicles without the intervention of other tissues. The separation period was formed within 60 to 80 days after formation of skin organoids in the case of the hair germ stage, while separation was carried out with those formed within 80 to 100 days in the case of the hair peg stage (FIGS. 4A and 4B).

When hair follicles after the hair peg stage were separated and cultured in vitro, the fine structural differentiation of the hair follicles was subjected to H&E and immunofluorescence staining for verification (FIG. 4C).

### 2. Optimization of conditions of the hair follicle culture support

After producing an artificial extracellular matrix, a coating support on a transwell culture insert used to culture hair follicles, under various conditions, hair follicles were cultured at the air-liquid interface. Hair follicles after the hair germ stage were separated and cultured on collage-only, Matrigel-only, and an extracellular matrix mixture containing collagen and Matrigel.

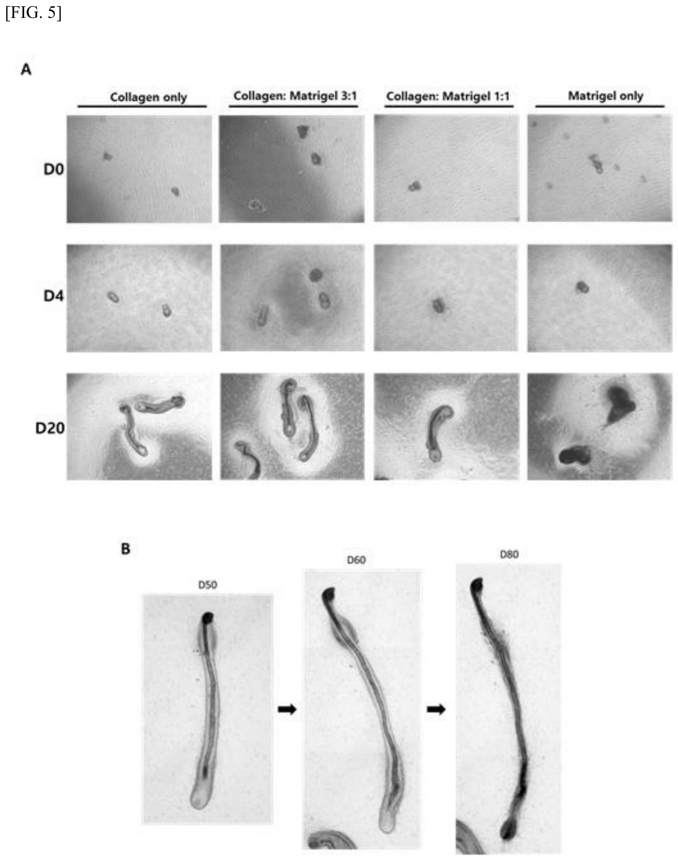

As a result, it was found that hair follicles grew when cultured under collagen-only conditions, but the differentiation into hair and growth rate were slower than under conditions where an extracellular matrix including collagen and Matrigel was mixed, with a discovery that hair follicles clumped together and failed to be cultured when cultured under Matrigel-only conditions. Finally, it was found that the growth rate of hair follicles and hair was most enhanced in the extracellular matrix containing collagen or a mixture of collagen and Matrigel (collagen:Matrigel = 3:1 to 1:1 ratio in collagen and Matrigel mixed culture) (FIG. 5A). In addition, it was determined that hair follicles could be cultured in vitro for more than 60 days on an extracellular matrix mixture containing collagen and Matrigel, and the growth of hair follicles stopped after 80 days (FIG. 5B).

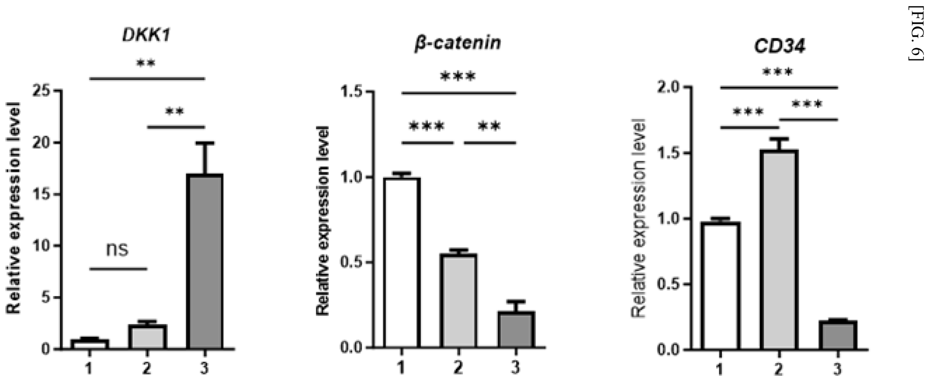

Hair follicles were sorted within 10 days, 30 to 50 days, and more than 60 days after hair follicle culture, and expression of genes related to hair growth induction and inhibition was observed via quantitative PCR. Expression of the DKK-1 gene, an alopecia-inducing cytokine, was significantly increased in hair follicles older than 60 days, while that of β-catenin, a gene involved in hair growth, as well as CD34, a major marker of hair follicle stem cells, was decreased. This showed that the growth and degeneration of hair follicles may be observed even under in vitro culture conditions (FIG. 6).

### <Example 3> Observation of growth of in-vivo hair follicles during transplantation

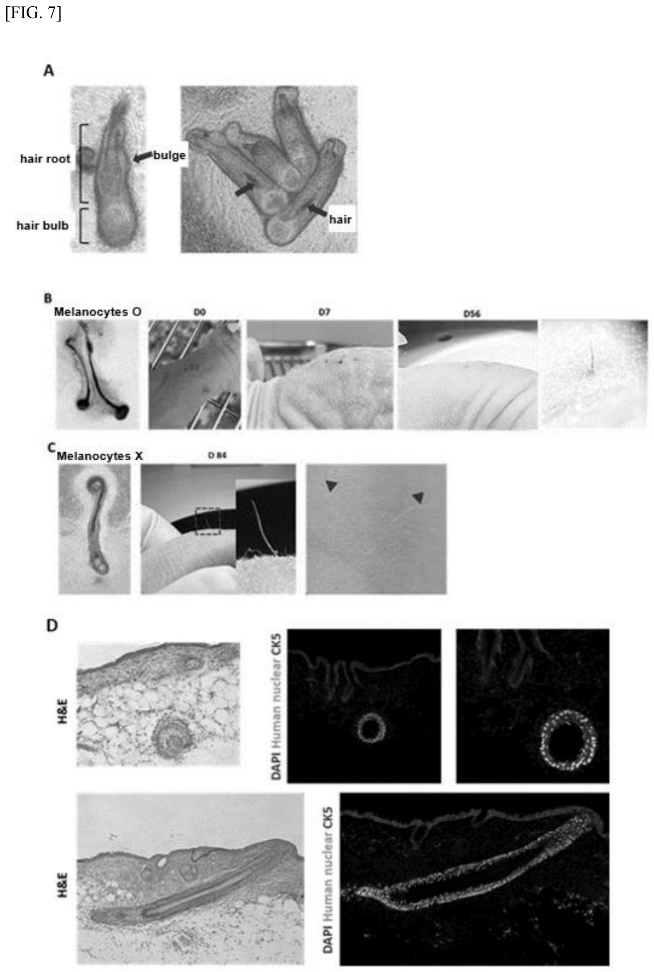

Hair follicles used for transplantation were those that the morphological structure of the hair follicles could be distinguished into the hair bulb and hair root parts after in vitro culture, differentiation into the bulge part was clearly progressed, and hair began to be produced (FIG. 7A).

A shallow puncture wound was made on the skin of 6-week-old Balb/c nu/nu mice with a 20G injection needle, approximately 0.5 mm vertically and 2.5 mm horizontally, almost parallel to the surface, and hair follicle organoids were transplanted intradermally into the wound site. Thereby, it was attempted to identify its potential as a transplant material.

As a result, the existing hair disappeared first within a week, and then new hair was observed about a month later. Growth of white or black hair was observed depending on the presence of melanocytes in the hair follicle organoids (FIGS. 7B, 7C).

The hairs were found to originate from human, not mouse, through H&E staining and immunofluorescence staining using human nuclear antigen antibodies (FIG. 7D). This is significant in that the produced hair follicle organoids allowed the growth of hair through the skin layer of hair-bearing mice even after transplantation, indicating that hair follicle organoids are able to function as a potential hair transplant material.

### <Example 4> Identification of potential for drug screening

The evaluation of the efficacy of alopecia promotion and alleviation of alopecia symptoms is mainly conducted through animal experiments, and in vitro testing methods have not yet been established, where existing known in vitro testing methods have limitations in evaluating the growth and morphology of hair follicles and studying signal transmission mechanisms, such as treatment of hair follicle papilla cells.

In the present disclosure, since the morphological and gene expression changes of the hair follicles were observed according to growth, it was determined that evaluation of various effective substances was possible.

To determine the possibility of evaluating effective substances, in addition to a group treated with 10⁻⁵M dihydrotestosterone (DHT), a hormone well known to act on hair follicles and cause alopecia, treatment with 10uM minoxidil (MXD), known for its hair growth effect, was performed simultaneously with DHT for 10 days.

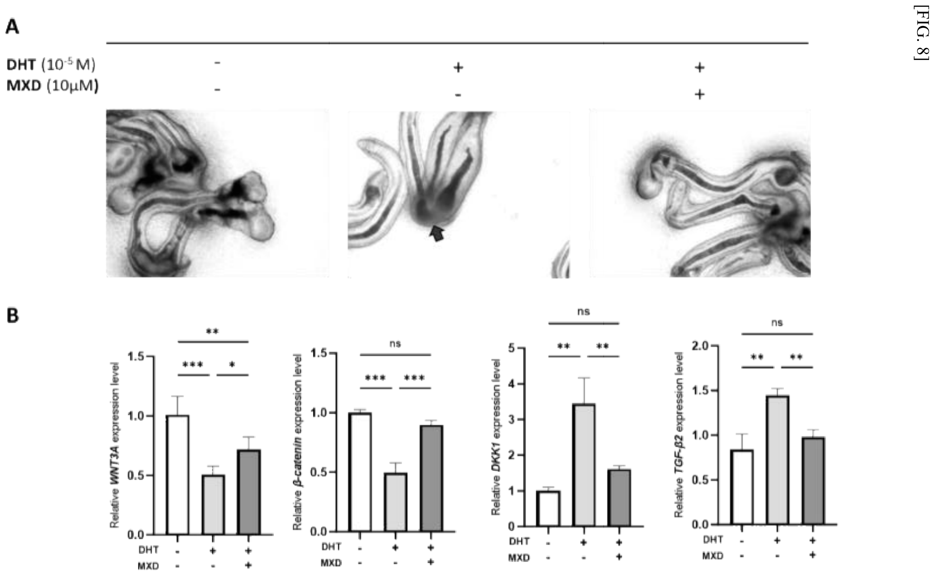

As a result, in terms of morphology, in the DHT-only treatment group, the length of the hair follicles was shorter, and the boundary of the hair papilla cell aggregates was blurred and damaged, whereas in the MXD co-treatment group, the length of the hair follicles was longer, and the boundary of the hair papilla cell aggregates was clearer, with a relatively reduced degree of damage compared to the DHT-only group (FIG. 8A).

Expression of genes related to promotion or inhibition of hair follicle growth was detected via quantitative PCR. As a result, in the DHT-only group, expression of WNT3A and β-catenin, which are involved in promoting hair follicle growth, decreased compared to the control group, whereas it significantly increased in the MXD co-treatment group. In addition, in the DHT-only group, expression of DKK-1 and TGF-β2, which are involved in inhibiting hair follicle growth, significantly increased compared to the control group, and significantly decreased in the MXD co-treatment group. It was determined that an alopecia-like reaction may occur in hair follicle organoids by DHT, and symptoms of alopecia were alleviated by MXD (FIG. 8B).

In addition to male hormones, factors that may cause alopecia (ultraviolet rays or stress hormones) may also be used to evaluate effective substances for a hair growth promoter, anti-alopecia agent, or alopecia treatment agent by producing an alopecia-mimicking model.

Depending on the order of treatment of the effective substance and the alopecia-inducing substance, the target of drug screening may be diverse, wherein it may be possible to identify the effect of preventing and protecting alopecia when the effective substance is treated first as well as the hair growth promoting and therapeutic effects when treated later, and, not limited thereto, even when various substances are treated alone to normal hair follicle organoids, the substance may be used as a platform to allow identification of the effect of inhibiting or promoting hair follicle growth.

Therefore, by determining that the efficacy of the substance identified thereby may be evaluated by the hair follicle, it was noticed that evaluation of other effective substances is also possible, and that not only a single hair follicle but also a strip-like hair follicle organoid may be utilized to evaluate the effect of a drug on multiple hair follicles.

The spherical skin organoid was planar-sectioned and spread out during a period of formation within 60 to 80 days at the hair germ stage or within 80 and 100 days at the hair peg stage, followed by vertical cutting at 1 mm intervals.

The cut sections were placed face down and upward on an artificial extracellular matrix consisting of collagen only or a mixture of collagen and Matrigel in a ratio of 1:10 to 10:1 as a coating support on a transwell culture insert, followed by culture at the air-liquid interface.

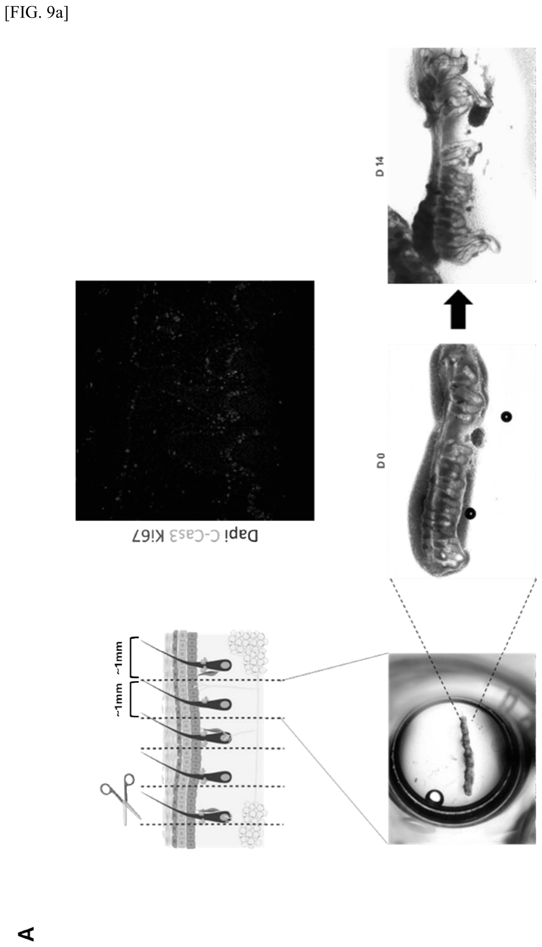

When the hair follicle strips thus cultured were subjected to immunofluorescence staining for identification of apoptosis (cleaved caspase-3) and cell proliferation (Ki-67), it was determined that proliferation was possible without cell death (FIG. 9A).

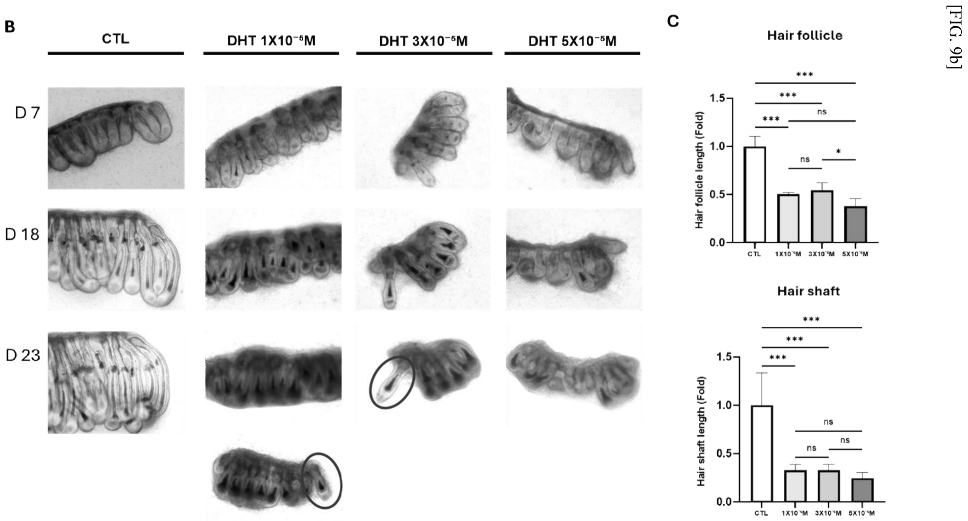

After 3 days of culture using strip-shaped hair follicle organoids, it was observed that the length of hair follicles and hair was reduced, and growth was inhibited when treated with alopecia-inducing hormone (DHT). Additionally, it was determined that the hair follicles were atrophied, and the hair papilla cells and the hair follicles began to separate (FIGS. 9B, 9C).

Thereby, it was found that strip-like hair follicle organoids may also be utilized to evaluate the effective substances for a hair growth promoter, anti-alopecia agent, or alopecia treatment agent.

The foregoing description herein is for illustrative purposes only, and it will be appreciated by those skilled in the art to which the present disclosure pertains that the present disclosure may be easily modified into other specific forms without altering the technical idea or essential features of the present disclosure. Therefore, it should be understood that the examples set forth above are exemplary in all respects and not limiting.

The scope of the present disclosure is indicated by the appended claims, and all changes or modifications derived from the meaning and scope of the claims and their equivalent concepts should be construed as being included in the scope of the present disclosure.

## Claims

1. A method of preparing a hair follicle organoid, the method comprising:
(1) separating hair follicles from a skin organoid; and
(2) culturing the separated hair follicles at an air-liquid interface.

2. The method of claim 1, wherein the skin organoid in step (1) is prepared by:
(a) culturing a pluripotent stem cell-derived organoid in the presence of a Wnt agonist;
(b) culturing the culture of step (a) in a medium for skin organoid maturation for at least 40 days; and
(c) cutting the culture of step (b) followed by culture at an air-liquid interface.

3. The method of claim 2, wherein the Wnt agonist is added on day 5 to day 7 of culturing the pluripotent stem cells.

4. The method of claim 1, wherein the skin organoid expresses one or more selected from the group consisting of keratin 5 (KRT5), KRT10, KRT15, KRT17, loricrin, filaggrin, SRY-box transcription factor 2 (SOX2), and Melan-A.

5. The method of claim 1, wherein the hair follicle of step (1) comprises any one or more selected from the group consisting of a dermal sheath, an outer sheath, an inner sheath, a hair papilla cell, a hair follicle cell, a bulge area, and a sebaceous gland.

6. The method of claim 1, wherein the hair follicles of step (1) are separated at a hair germ stage or a hair peg stage.

7. The method of claim 6, wherein the hair germ stage is formed within 60 to 80 days after formation of a skin organoid.

8. The method of claim 6, wherein the hair peg stage is formed within 80 to 100 days after formation of a skin organoid.

9. The method of claim 1, wherein the separation in step (1) is performed by microdissection.

10. The method of claim 1, wherein the culturing in step (2) is performed on a culture insert.

11. The method of claim 10, wherein the culture insert is coated with collagen and Matrigel; or collagen.

12. The method of claim 11, wherein the collagen and Matrigel are in a ratio of 1:10 to 10:1.

13. A hair follicle organoid prepared by the preparation method according to claim 1.

14. A hair transplant material comprising the hair follicle organoid according to claim 13.

15. A method of preparing a strip-like hair follicle organoid, the method comprising:
(1) planar sectioning a skin organoid and spreading it, followed by vertical cutting; and
(2) culturing the cut skin organoid at an air-liquid interface.

16. The method of claim 15, wherein the skin organoid of step (1) is prepared by:
(a) culturing a pluripotent stem cell-derived organoid in the presence of a Wnt agonist;
(b) culturing the culture of step (a) in a medium for skin organoid maturation for at least 40 days; and
(c) cutting the culture of step (b) followed by culture at air-liquid interface.

17. The method of claim 15, wherein the cutting in step (1) is performed when hair follicles in the skin organoid are in a hair germ stage or a hair peg stage.

18. The method of claim 15, wherein the cutting in step (1) is performed by cutting the spread skin organoid at intervals of 1 mm to 3 mm.

19. A strip-like hair follicle organoid prepared by the preparation method according to claim 15.

20. A hair transplant material comprising the strip-like hair follicle organoid according to claim 19.

21. A method of preparing a hair follicle organoid for an alopecia-mimicking model, the method comprising:
(1) separating hair follicles from a skin organoid;
(2) culturing the separated hair follicles at an air-liquid interface to produce a hair follicle organoid; and
(3) treating the hair follicle organoid with an alopecia-inducing factor.

22. The method of claim 21, wherein the alopecia-inducing factor in step (3) is male hormones, stress hormones, or ultraviolet rays.

23. The method of claim 22, wherein the hormone is dihydrotestosterone, testosterone, or thyroid hormone.

24. The method of claim 22, wherein the stress hormone is cortisol.

25. A hair follicle organoid for an alopecia-mimicking model produced by the preparation method according to claim 21.

26. The hair follicle organoid of claim 25, wherein the hair follicle organoid for the alopecia-mimicking model has any one or more characteristics selected from the group consisting of:
1) reduction in a length of hair follicles; 2) damage to hair papillary cell aggregates; 3) decreased expression of WNT3A or β-catenin; or 4) increased expression of DKK-1 or TGF-β2.

27. A method of preparing a strip-like hair follicle organoid for an alopecia-mimicking model, the method comprising:
(1) planar sectioning a skin organoid and spreading it, followed by vertical cutting;
(2) culturing the cut skin organoid at an air-liquid interface to produce a strip-like hair follicle organoid; and
(3) treating the strip-like hair follicle organoid with an alopecia-inducing factor.

28. A strip-like hair follicle organoid for an alopecia-mimicking model produced by the preparation method according to claim 27.

29. A method of screening a hair growth promoter, anti-alopecia agent, alopecia relief agent, or alopecia treatment agent, the method comprising:
(1) separating hair follicles from a skin organoid;
(2) culturing the separated hair follicles at an air-liquid interface to produce a hair follicle organoid;
(3) treating the hair follicle organoid with an alopecia-inducing factor to produce a hair follicle organoid for an alopecia-mimicking model; and
(4) treating the hair follicle organoid for the alopecia-mimicking model with a candidate substance for a hair growth promoter, anti-alopecia agent, alopecia relief agent, or alopecia treatment agent.

30. The method of claim 29, wherein the method comprises, if expression of WNT3A or β-catenin increases or expression of DKK-1 or TGF-β2 decreases after treatment of the candidate substance in step (4), determining the candidate substance as a hair growth promoter, anti-alopecia agent, alopecia relief agent, or alopecia treatment agent.

31. A method of screening a hair growth promoter, anti-alopecia agent, alopecia relief agent, or alopecia treatment agent, the method comprising:
(1) planar sectioning a skin organoid and spreading it, followed by vertical cutting;
(2) culturing the cut skin organoid at an air-liquid interface to produce a strip-like hair follicle organoid;
(3) treating the strip-like hair follicle organoid with an alopecia-inducing factor to produce a strip-like hair follicle organoid for an alopecia-mimicking model; and
(4) treating the strip-like hair follicle organoid for the alopecia-mimicking model with a candidate substance for a hair growth promoter, anti-alopecia agent, alopecia relief agent, or alopecia treatment agent.

32. The method of claim 31, wherein the method comprises, if expression of WNT3A or β-catenin increases or expression of DKK-1 or TGF-β2 decreases after treatment of the candidate substance in step (4), determining the candidate substance as a hair growth promoter, anti-alopecia agent, alopecia relief agent, or alopecia treatment agent.

33. A method of screening a hair growth promoter, anti-alopecia agent, alopecia relief agent, or alopecia treatment agent, the method comprising:
(1) separating hair follicles from a skin organoid;
(2) culturing the separated hair follicles at an air-liquid interface to produce a hair follicle organoid; and
(3) treating the hair follicle organoid with a candidate substance for a hair growth promoter, anti-alopecia agent, alopecia relief agent, or alopecia treatment agent.

34. The method of claim 33, wherein the method comprises, if expression of WNT3A or β-catenin increases or expression of DKK-1 or TGF-β2 decreases after treatment of the candidate substance in step (3), determining the candidate substance as a hair growth promoter, anti-alopecia agent, alopecia relief agent, or alopecia treatment agent.

35. A method of screening a hair growth promoter, anti-alopecia agent, alopecia relief agent, or alopecia treatment agent, the method comprising:
(1) planar sectioning a skin organoid and spreading it, followed by vertical cutting;
(2) culturing the cut skin organoid at an air-liquid interface to produce a strip-like hair follicle organoid; and
(3) treating the strip-like hair follicle organoid with a candidate substance for a hair growth promoter, anti-alopecia agent, alopecia relief agent, or alopecia treatment agent.

36. The method of claim 35, wherein the method comprises, if expression of WNT3A or β-catenin increases or expression of DKK-1 or TGF-β2 decreases after treatment of the candidate substance in step (3), determining the candidate substance as a hair growth promoter, anti-alopecia agent, alopecia relief agent, or alopecia treatment agent.

37. A method of screening a cosmetic composition for promoting hair growth, preventing alopecia, or alleviating alopecia, the method comprising:
(1) separating hair follicles from a skin organoid;
(2) culturing the separated hair follicles at an air-liquid interface to produce a hair follicle organoid;
(3) treating the hair follicle organoid with an alopecia-inducing factor to produce a hair follicle organoid for an alopecia-mimicking model; and
(4) treating the hair follicle organoid for the alopecia-mimicking model with a candidate substance for a cosmetic composition for promoting hair growth, preventing alopecia, or alleviating alopecia.

38. A method of screening a cosmetic composition for promoting hair growth, preventing alopecia, or alleviating alopecia, the method comprising:
(1) planar sectioning a skin organoid and spreading it, followed by vertical cutting;
(2) culturing the cut skin organoid at an air-liquid interface to produce a strip-like hair follicle organoid;
(3) treating the strip-like hair follicle organoid with an alopecia-inducing factor to produce a stirp-like hair follicle organoid for an alopecia-mimicking model; and
(4) treating the stirp-like hair follicle organoid for the alopecia-mimicking model with a candidate substance for a cosmetic composition for promoting hair growth, preventing alopecia, or alleviating alopecia.

39. A method of screening a cosmetic composition for promoting hair growth, preventing alopecia, or alleviating alopecia, the method comprising:
(1) separating hair follicles from a skin organoid;
(2) culturing the separated hair follicles at an air-liquid interface to produce a hair follicle organoid; and
(3) treating the hair follicle organoid with a candidate substance for a cosmetic composition for promoting hair growth, preventing alopecia, or alleviating alopecia.

40. A method of screening a cosmetic composition for promoting hair growth, preventing alopecia, or alleviating alopecia, the method comprising:
(1) planar sectioning a skin organoid and spreading it, followed by vertical cutting;
(2) culturing the cut skin organoid at an air-liquid interface to produce a strip-like hair follicle organoid; and
(3) treating the strip-like hair follicle organoid with a candidate substance for a cosmetic composition for promoting hair growth, preventing alopecia, or alleviating alopecia.
